(19) Europäisches Patentamt
European Patent Office
Office européen des brevets

(11) EP 1 529 543 A1

(12) # DEMANDE DE BREVET EUROPEEN

(43) Date de publication:
**11.05.2005 Bulletin 2005/19**

(51) Int Cl.[7]: **A61L 27/46**, A61L 27/50

(21) Numéro de dépôt: **03292759.2**

(22) Date de dépôt: **04.11.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(71) Demandeurs:
- **INSTITUT NATIONAL DE LA SANTE ET DE LA RECHERCHE MEDICALE (INSERM)**
  **75013 Paris (FR)**
- **L'UNIVERSITE DE NANTES**
  **44000 Nantes (FR)**

(72) Inventeurs:
- **Weiss, Pierre**
  **44800 Saint Herblain (FR)**
- **Guicheux, Jerôme**
  **44000 Nantes (FR)**
- **Daculsi, Guy**
  **44360 Vigneux de Bretagne (FR)**
- **Grimandi, Gael**
  **44000 Nantes (FR)**
- **Vinatier, Claire**
  **44300 Nantes (FR)**

(74) Mandataire: **Bernasconi, Jean Raymond et al**
**c/o Cabinet Lavoix,**
**2, Place d'Estienne d'Orves**
**75441 Paris Cedex 09 (FR)**

(54) **Utilisation d'un hydrogel pour la culture de chondrocytes**

(57) L'invention concerne l'utilisation d'un hydrogel d'hydroxyéthylcellulose (HEC) silanisée ou d'hydroxypropylméthylcellulose (HPMC) silanisé, auto-réticulant en fonction du pH, pour la culture tridimensionnelle *ex vivo* de chondrocytes.

EP 1 529 543 A1

## Description

L'invention concerne l'utilisation d'un hydrogel cellulosique pour la culture tridimensionnelle de chondrocytes en vue de leur implantation en un site cartilagineux.

Le cartilage est un tissu conjonctif de soutien spécialisé non vascularisé, non innervé, résistant et élastique. Il est présent au niveau des côtes, du sternum, du nez et des oreilles mais également au niveau des articulations. Le cartilage contient des cellules spécialisées appelées chondrocytes et une matrice extracellulaire (MEC) composée essentiellement de fibres de collagène et de protéoglycanes.

Le cartilage articulaire peut être le siège de nombreuses altérations d'origine inflammatoire (polyarthrite rhumatoïde, ostéoarthrite), traumatologique ou liées au vieillissement (arthrose). L'établissement de ces atteintes cartilagineuses conduit, à plus ou moins long terme, à une dégradation de la matrice extracellulaire et une diminution de la cellularité. L'absence de vascularisation et de prolifération des chondrocytes confère à ce tissu de faibles capacités de réparation qui rendent ces processus cataboliques irréversibles. Au regard du vieillissement de la population, ces pathologies dégénératives concernent aujourd'hui une part importante de la population et représentent donc un enjeu majeur de santé publique. Dans ce contexte, la communauté scientifique s'intéresse depuis de nombreuses années aux moyens de régénérer un tissu cartilagineux fonctionnel.

Afin de stimuler les faibles propriétés de réparation spontanée du cartilage, des techniques chirurgicales de chondroplastie abrasive, de micro fracture ou de spongialisation ont été proposées (Hunziker, 2002). Ces techniques, permettant la formation d'un caillot sanguin d'origine sous-chondrale, conduisent à la formation d'un tissu cartilagineux qui demeure fibreux et transitoire (Shapiro et al., 1993). Parallèlement, des techniques de transplantation de tissu possédant des propriétés chondrogéniques ont également été étudiées. L'utilisation de transplants autologues de périoste ou de périchondre et de tissus ostéochondral (mosaicplastie) a permis la néoformation d'un tissu de type cartilagineux après implantation (Hunziker, 2002). Les nombreuses limitations de ces techniques (instabilité de la greffe, calcification, faible reproductibilité des résultats cliniques) et la restriction de leur indication à la réparation de lésions focales, ont récemment conduit au développement de nouvelles techniques d'ingénierie tissulaire appliquées aux atteintes traumatologiques du cartilage.

Brittberg et al. furent les premiers à proposer une méthode de réparation basée sur la transplantation de chondrocytes autologues (Brittberg et al., 1994). Celle-ci se déroule en trois temps, tout d'abord un fragment de cartilage est prélevé dans une zone non portante afin d'isoler des chondrocytes. Ceux-ci sont alors multipliés *in vitro* en monocouche puis réimplantés sous un lambeau périosté au niveau de la lésion. Les résultats ont montré une réparation du défaut cartilagineux, mais les patients doivent subir deux interventions chirurgicales lourdes. Par ailleurs, le phénotype des cellules après leur amplification en monocouche reste à déterminer.

Plusieurs matrices tridimensionnelles d'origine végétale ou animale ont par ailleurs été développées comme les éponges de collagène, la fibrine, l'acide hyaluronique ou l'alginate (Cancedda et al., 2003). Le risque de transmission virale associé à l'utilisation de ces matériaux biologiques et les réactions inflammatoires observées lors des expérimentations pré-cliniques, ont dirigé les recherches actuelles vers le développement de biomatériaux totalement synthétiques (Hunziker, 2002). Des polymères tels que l'acide poly lactique, l'acide poly glycolique, les fibres de carbone, le polyesteruréthane, le Dacron et le Téflon ont été proposés et utilisés *in vitro* pour la culture 3D de chondrocytes ou de cellules mésenchymateuses. Parmi ces polymères synthétiques, certains ont entraîné des réactions immunitaires ou une inflammation après leur implantation en site cartilagineux (Cancedda et al., 2003).

Des compositions d'hydrogel injectables sont décrites notamment dans le brevet US 6,129,761.

Les inventeurs ont maintenant mis en évidence l'intérêt de cultiver des chondrocytes en trois dimensions au sein d'un hydrogel auto-réticulant en fonction du pH, en vue d'une l'implantation dans un site cartilagineux.

L'hydrogel utilisé est un hydrogel auto-réticulant en fonction du pH, qui est constitué d'une solution aqueuse d'hydroxyéthylcellulose (HEC) ou d'hydroxypropylméthylcellulose (HPMC) sur laquelle sont greffés des groupements silanes qui permettent la formation de liaisons covalentes entre les chaînes d'HEC ou d'HPMC.

Ce type de matériau polymérique est décrit en association avec une charge minérale dans la demande de brevet internationale WO 97/05911.

Ce matériau est constitué de préférence d'un polymère de formule simplifiée :

$$(HEC\ ou\ HPMC)\text{-}O\text{-}X\text{-}Si(OZ)_3$$

pouvant être obtenu par réaction d'hydroxyéthylcellulose (HEC) ou d'hydroxypropylméthylcellulose avec un composé de formule (1) :

$$X\,Si(OZ)_3 \qquad (1)$$

où X représente un atome d'halogène ou un groupe hydrocarboné à fonction époxy, notamment en $C_{2-20}$, et Z est choisi parmi un atome d'hydrogène, un métal alcalin et un groupe alkyle, notamment en $C_{1-5}$.

Le composé de formule (1) peut être par exemple le (3-glycidoxypropyl)triméthoxysilane

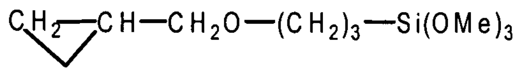

En milieu basique, le composé organosilicié se greffe sur l'HEC ou l'HPMC avec ouverture de l'époxyde et les groupes métoxysilane sont hydrolysés pour conduire à un polymère de formule simplifiée :

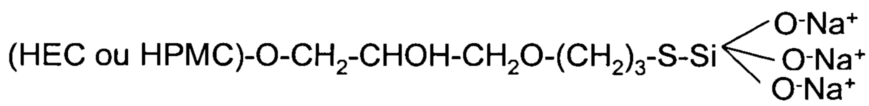

De préférence, le polymère est un polymère d'HPMC silanisée.

Ce polymère est stable en solution aqueuse à un pH supérieur ou égal à environ 12,4. Une acidification de la solution provoque une augmentation progressive de la viscosité et la formation d'un hydrogel. Ce phénomène physique correspond à la réticulation du polymère par (i) transformation des groupes silanolates en groupes silanols :

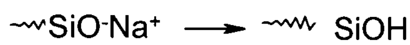

puis, formation d'un réseau tridimensionnel par

(ii) condensation d'un silanol sur un autre silanol

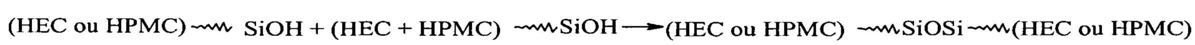

et/ou

(iii) condensation d'un silanol sur un groupe hydroxy des cycles des éthers de cellulose ou des substituants

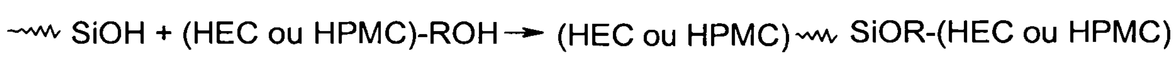

Cette réticulation de type covalent provoquée par une baisse du pH de la solution aqueuse du polymère est réversible et l'hydrogel se redissout lorsque l'on augmente le pH du milieu. Ainsi, avant d'être utilisé, le polymère synthétisé peut se présenter sous la forme d'une poudre qui peut être dissoute dans une solution alcaline d'hydroxyde de sodium. Le pH de gélification se situe entre 7 et 12 en fonction de la vitesse de réticulation recherchée.

Le gel obtenu est stérilisable à l'autoclave (par exemple à 121°C pendant 20 minutes).

Le polymère en solution aqueuse basique, avant réticulation, peut également être mélangé à une solution tampon physiologique avant son utilisation biologique. Le pH du mélange final est ainsi adapté au temps de manipulation recherché avant réticulation de l'ensemble. Ce temps de réticulation est mesurable par rhéométrie oscillatoire et peut varier de 5 minutes à 5 jours en fonction des paramètres de ce mélange, principalement le pH et la température (Bourges et al., 2002a ; Bourges et al., 2002b).

Pour fournir un taux de réticulation convenable pour l'application envisagée, il est souhaitable que les groupes latéraux porteurs de silicium, de type silanolate ou précurseur de silanolate de métal alcalin ou d'ammonium, représentent de 0,5 à 5% du poids sec total du polymère.

L'invention a pour objet l'utilisation d'un tel hydrogel d'HEC silanisée ou d'HPMC silanisée, auto-réticulant en fonction du pH, pour la culture tridimensionnelle *ex vivo* de chondrocytes.

L'hydrogel utilisé présente notamment les avantages suivants :

- il est biocompatible avec des cellules chondrocytaires intégrées en son sein ;
- les chondrocytes sont intégrés de manière intime dans l'hydrogel ;
- les chondrocytes dédifférenciés par amplification en monocouche se redifférencient au sein de l'hydrogel, et maintiennent des caractéristiques de chondrocytes ;
- la réticulation est contrôlable par le pH du tampon biologique ajouté au mélange d'hydrogel et de cellules chondrocytaires.

Les chondrocytes mis en culture dans l'hydrogel sont de préférence des chondrocytes humains ou animaux, de préférence des chondrocytes autologues, de patients présentant une lésion du cartilage. Il peut par exemple s'agir de cellules prélevées au niveau d'une partie du cartilage articulaire ou au niveau du cartilage nasal.

L'invention a également pour objet un procédé *ex vivo* de préparation d'un complexe de cellules intégrées dans un hydrogel, complexe destiné à être injecté dans un site cartilagineux, ledit procédé comprenant le mélange *ex vivo* de chondrocytes avec un hydrogel d'HEC ou d'HPMC silanisée, autoréticulant en fonction du pH, dans un tampon biologique au pH approprié pour la réticulation de l'hydrogel, dans des conditions et une durée appropriées pour l'intégration et la culture tridimensionnelle des chondrocytes dans l'hydrogel.

Tout tampon biologique peut être utilisé par l'homme du métier. A titre d'exemples, on peut citer le tampon phosphate (PBS, phosphate buffered salt), tampon HEPES, ou TRIS. Tout milieu biologique connu de l'homme du métier est en outre utilisable, tel que le milieu DMEM ou alpha-MEM (alpha minimum essential medium).

De manière préférée, le procédé comprend les étapes *ex vivo* suivantes :

- l'amplification de chondrocytes en monocouche, sur un support solide, tel qu'une plaque de culture ;

- la récolte des chondrocytes amplifiés, dédifférenciés de par leur amplification en monocouche ;

- le mélange des chondrocytes amplifiés dédifférenciés avec l'hydrogel dans un tampon biologique au pH approprié pour la réticulation de l'hydrogel, ce qui conduit à l'intégration des chondrocytes au sein de l'hydrogel et à leur redifférenciation.

Les propriétés rhéologiques de l'hydrogel composé d'HEC ou d'HPMC silanisée permettent son injection sur le lieu d'implantation, favorisant ainsi la vectorisation des chondrocytes par chirurgie peu invasive.

L'hydrogel au sein duquel les chondrocytes ont été cultivés peut être implanté dans un site de la lésion, normalement occupé par du cartilage.

Les lésions visées peuvent être des pertes de substances cartilagineuses focales liées à des séquelles traumatologiques ou, plus généralement, toutes les atteintes ou pertes cartilagineuses ostéoarticulaires ou plastiques.

L'invention a donc également pour objet un procédé du traitement du corps humain ou animal, comprenant l'administration par injection d'hydrogel tel que défini plus haut préalablement colonisé *ex vivo* par des chondrocytes, par exemple selon le procédé décrit plus haut.

L'injection peut être réalisée à l'aide d'un système comportant une seringue stérilisable et des embouts munis de pistons à usage unique, par exemple le système commercialisé par HAWE NEOS DENTAL, comprenant une seringue stérilisée à l'autoclave (réf. N° 440, Seringue Hawe-Centrix C-R$^{R}$, Mark III) et des embouts (réf. N° 445).

Les figures et exemples suivants illustrent l'invention sans en limiter la portée.

**LEGENDE DES FIGURES** :

Les Figures 1A à 1C représentent l'activité MTS des chondrocytes cultivés pendant 24, 48 et 72 heures soit dans les conditions contrôles (absence d'hydrogel), soit au contact de l'hydrogel si-HPMC, soit en présence d'actinomycine D (5 µg/ml). SW1353 (Fig. 1A), C28/12 (Fig. 1B) et chondrocytes nasaux humains (Fig. 1C).
*p<0,001 comparé au contrôle à chaque temps.

Les Figures 2A à 2C représentent le dénombrement des chondrocytes cultivés pendant 24, 48 et 72 heures soit dans les conditions contrôles (absence d'hydrogel), soit au contact de l'hydrogel si-HPMC, soit en présence d'actinomycine D (5 µg/ml). SW1353 (Fig. 2A), C28/12 (Fig. 2B) et chondrocytes nasaux humains (Fig. 2C).
*p<0,001 comparé au contrôle à chaque temps.

**EXEMPLES :**

**Exemple 1 : préparation de l'hydrogel**

*Matériels :*

- HPMC E4M® (Colorcon- Dow Chemical, France)
- Glycidoxypropyltriméthoxysilane (GPTMS) (Acros, Belgique)
- HEPES (Sigma-Aldrich, St Louis, USA)
- NaOH (VWR International, Fontenay-sous-Bois, France)
- NaCl (VWR International)
- HCl 0.1 M (Sigma Aldrich).
- Sérum de veau foetal (SVF) (Dominique Dutscher, Brumath, France).

a) Synthèse de la poudre d'hydroxypropyl méthylcellulose silanisée (Si-HPMC)

Les synthèses sont réalisées sur des quantités de 240 grammes d'HPMC. Le polysaccharide sélectionné est l'E4M®. La synthèse est réalisée avec du glycidoxypropyltriméthoxysilane (GPTMS) dans un ballon de 6 litres en milieu hétérogène dans un solvant organique (Bourges et al., 2002). La synthèse est effectuée sous ébullition pendant 3 heures. La poudre d'HPMC silanisée est séchée une première fois à l'étuve pendant une nuit puis lyophilisée (Christ Alpha 1-4 ST).

b) Préparation de la solution d'hydroxypropyl méthylcellulose silanisée

Six grammes de poudre de Si-HPMC sont solubilisés dans 200 ml d'une solution de NaOH à 0.2 M. Ce mélange est agité pendant 48 heures. La solution de Si-HPMC est ensuite dialysée pendant 16h dans une solution de NaOH 0.09 M. Puis une deuxième dialyse est réalisée dans une solution de NaOH 0.09 M pendant 2 heures. Cette solution de Si-HPMC à 3% est ensuite aliquotée et stérilisée à la vapeur (121°C, 30 minutes).

c) Induction de la réticulation de l'hydrogel

L'hydrogel utilisé pour la culture cellulaire est préparé extemporanément en mélangeant, sous hotte à flux laminaire, 7.5 ml de solution de Si-HPMC avec 7.5 ml de tampon HEPES et supplémenté de 10% de SVF. Le tampon HEPES est préparé par dissolution de 3.1 g d'HEPES et 0.8g de NaCl dans une solution d'HCl à 0.03M. Cette solution est ensuite aliquotée et stérilisée à la vapeur (121°C, 30 minutes).

Ce mélange HEPES/Si-HPMC est agité au Vortex 15 secondes puis centrifugé 2 minutes à 1200 rotations par minutes (rpm). Ce mélange est transféré en plaque de culture. Le milieu de culture est ajouté 1 heure après.

d) Caractérisation physique de l'hydrogel par Rhéologie

*Matériels et méthodes*

Afin de caractériser la réticulation de l'hydrogel, les inventeurs ont effectué la mesure de deux paramètres rhéologiques: le module dispersif G" (caractéristique des liquides) et le module conservatif G' (caractéristique des solides). Cette mesure est réalisée avec un rhéomètre (Rhéostress 300, Thermo Haake, Karlsruhe, Germany) équipé d'un cône plan de 60 mm de diamètre et de 1° d'angle (Cône plan C60/1 titan, Thermo Haake). L'entrefer entre la troncature et le plateau est de 0.053 mm. La mesure est réalisée en mode oscillatoire avec trois fréquences d'oscillations (1 Hz, 3.2 Hz et 10 Hz) et une contrainte imposée de 1 Pascal, à une température de 37°C et pendant 1 200 000 secondes (13.8jours). Les résultats sont exprimés en Pa.

*Résultats*

L'hydrogel présente les caractéristiques d'un liquide visqueux au moment de sa préparation. Lors de sa réticulation, ses caractéristiques physiques évoluent vers celles d'un gel. L'hydrogel préparé selon le mode de réalisation préféré, présenté à l'exemple 1, se compose, une fois la réticulation terminée (10 jours), de 1,5 % de polymère sec et de 98,5 % d'eau, une teneur en eau comparable à celle du cartilage. La taille calculée de la maille de l'hydrogel (0,22

µm) apparaît inférieure à la taille d'une cellule.

Dans le but de connaître les caractéristiques physiques de l'hydrogel, comme le point de gel qui correspond au début de la réticulation, le temps de réticulation et la taille de la maille de l'hydrogel, l'évolution en fonction du temps de la composante liquide (G") et de la composante élastique (G') a été mesurée, et ce, pour trois fréquences différentes. Donc à chaque fréquence correspond un couple G'/G". La TanΔ de chaque couple a été tracée, ces trois courbes se rejoignent en un point : le point de gel. La réticulation de l'hydrogel a débuté 33 minutes après sa préparation, et s'est achevée 244 heures plus tard (10 jours) moment où la composante élastique (G') atteint un plateau pour une valeur de 310 Pa. Cette valeur de la composante élastique nous a permis de calculer la taille de la maille de l'hydrogel.

$$\xi = \left(\frac{KT}{G'}\right)^{1/3} = \left(\frac{1.38\times10^{-23}\times300}{310}\right)^{1/3} = \left(\frac{4,14\times10^{-21}}{310}\right) = 23.7\,nanomètres$$

$K = 1.38.10^{-23}$
$T = 300$

Dans les conditions de préparation décrites ci-dessus, la taille statistique moyenne de la maille de l'hydrogel (Xi) était de 0,023 µm.

Tableau 1 :

| Résultats de la caractérisation rhéologique de l'Hydrogel de Si-HPMC à 3% | | | | | | | | |
|---|---|---|---|---|---|---|---|---|
| Vol de solution de Si-HPMC | Vol Tampon | % Si-HPMC | % final de Si-HPMC | pH mélange | G' | KT/G' | **Xi (ξ) en micromètre** | **Point de gel en minutes** |
| 1 | 1 | 3,00% | 1,50% | 7,37 | 310 | 1,3355E-23 | **0,023** | **33** |

**<u>Exemple 2 :</u> Test de cytotoxicité de l'hydrogel**

**<u>MATERIELS ET METHODES</u>**

**2.1 Culture cellulaire**

a) Lignées chondrocytaires

Matériels:

- Dulbecco's modified Eagle medium (DMEM) (Invitrogen corporation, Paisley, UK).
- Milieu nutritif Ham's F12 (Invitrogen corporation).
- Pénicilline/streptomycine (Invitrogen corporation)
- L-glutamine (Invitrogen corporation)
- Trypsine/EDTA (Invitrogen corporation).

Les inventeurs ont utilisé les lignées de chondrocytes humains SW1353 et C28/12 respectivement dérivées d'un chondrosarcome humain (Mengshol et al., 2000) et de cartilage costal humain immortalisé par l'antigène T du virus simien 40 (Goldring et al., 1994). Ces cellules sont cultivées dans un mélange volume/volume de DMEM et Ham's F12 supplémenté avec 10% de SVF, 1% de pénicilline/streptomycine et 1% de L-Glutamine (DMEM/F12 complet) dans une atmosphère humide à 37°C et 5 % CO2.

Le milieu de culture est renouvelé entièrement tous les deux jours. Lorsque les cellules atteignent 80-90 % de la confluence, elles sont traitées par 2 ml de Trypsine (0.025%) /EDTA (0.01 %). Après incubation 3 minutes à 37°C, la solution de trypsine/EDTA est collectée et centrifugée (8 min, 1200 rpm) en présence de 8 ml de milieux DMEM/F12 complet. Après élimination du surnageant, le culot cellulaire est reconstitué avec 10 ml de DMEM/F12 complet. Les cellules sont ensuite dénombrées et réparties dans des flacons de 25 $cm^2$ à une densité de 10 000 cellules/$cm^2$.

b) Chondrocytes primaires

- Isolement de chondrocytes nasaux humains

Matériels:

- Hank's balanced salt (HBSS, Invitrogen Corporation).
- Protéase (Pronase, Sigma-Aldrich).
- Collagénase type IV (Sigma-Aldrich).

Du cartilage nasal humain est prélevé après obtention du consentement éclairé de patients subissant une rhinoplastie reconstructrice. Le cartilage nasal est lavé cinq fois dans du Hank's Balanced Salts (HBSS) et découpé en copeaux sous hotte à flux laminaire. Les copeaux de cartilage sont ensuite incubés pendant 30 minutes à 37°C dans du HBSS contenant 1mg/ml de protéase. Les copeaux sont ensuite rincés trois fois dans du HBSS, puis incubés pendant 4 heures et sous agitation dans du DMEM complet (10 % SVF, 1% Pénicilline/Streptomycine et 1% L-Glutamine) contenant 0.625 mg/ml de collagénase. Le surnageant est centrifugé 5 minutes à 1400 rpm. Le culot est lavé une fois dans du HBSS, centrifugé (8 min, 1200 rpm) et resuspendu dans 10 ml de DMEM complet. Les cellules sont dénombrées et transférées en flacon de 25 $cm^2$ à une densité cellulaire de 10 000 cellules/$cm^2$. Les cellules sont maintenues en culture en atmosphère humide à 37°C et 5 % $CO_2$. Les milieux de culture sont renouvelés tous les deux jours. Lorsque les cellules atteignent 80-90 % de la confluence, elles sont traitées à la trypsine/EDTA comme décrit ci-dessus.

- Isolement de chondrocytes articulaires de lapin

Matériels:

- Hyaluronidase (Sigma-Aldrich).
- Trypsine (Sigma-Aldrich).
- collagénase type II de *Clostridium histolyticum* (290 U/mg, Sigma-Aldrich)
- Tamis cellulaire (Falcon, Franklin Lakes, USA).

Comme précédemment décrit par Ghayor et al., 2000, des lapins nouveaux-nés âgés de 12 ou 13 jours sont sacrifiés par dislocation cervicale après anesthésie. Sous hotte à flux laminaire, les pattes avant et arrière sont prélevées et débarrassées de leurs tissus mous. Les articulations des genoux et des épaules sont ensuite disloquées. A l'aide d'un scalpel, le cartilage articulaire est découpé en fines tranches et les copeaux obtenus sont placés dans du HBSS. Ces copeaux sont successivement incubés 10 minutes à 37°C dans 12 ml de HBSS contenant 0.05% de hyaluronidase, puis 15 minutes à 37°C dans 6 ml d'HBSS contenant 0.2% de trypsine. Après deux rinçages dans du HBSS, les copeaux sont transférés dans du HBSS à 0.2% de collagénase et incubés 30 minutes à 37°C.

Les copeaux subissent une dernière étape de digestion par incubation dans une solution de DMEM complet enrichi de 0.03% de collagénase pendant 15 heures. Le produit de digestion est filtré sur tamis cellulaire, collecté et centrifugé (8 min, 1200 rpm). Le culot cellulaire est resuspendu dans 20 ml de DMEM complet. Les cellules sont dénombrées et transférées en flacon de 25 $cm^2$ à une densité cellulaire de 10 000 cellules/$cm^2$. Les cellules sont finalement maintenues en culture en atmosphère humide à 37°C et 5 % $CO_2$. Les milieux de culture sont renouvelés tous les deux jours. Lorsque les cellules atteignent 80-90 % de la confluence, elles sont traitées à la trypsine/EDTA comme décrit ci-dessus.

**2.2. Etude de la cytotoxicité de l'hydrogel**

a) Conditions expérimentales

Matériels:

- Plaque de culture 24 puits Corning-Costar (Corning BV, Schiphol-Rijk, The Netherlands).
- Actinomycine D (Sigma-Aldrich)
- DMSO (Sigma-Aldrich)
- Méthyle Tetrazolium Salt (MTS) (Cell Titer 96 MTS, Promega corporation, Madison, WI).
- Phényle Méthyle Sulfoxyde (PMS) (Sigma-Aldrich).
- Phosphate buffered salt (PBS, Invitrogen corporation)

Afin de déterminer la cytotoxicité de l'hydrogel, les chondrocytes primaires humains, les cellules SW1353 et C28/I2, sont répartis dans des plaques de culture 24 puits à une densité de 10 000 cellules par cm2. Après 24 heures d'incubation à 37°C et 5% $CO_2$, les cellules sont cultivées en présence (500 µl de Si HPMC par puits) ou en absence d'hydrogel (500 µl de milieu complet) pendant 24, 48, et 72 heures. Des cellules cultivées en absence d'hydrogel sont également traitées par de l'Actinomycine D (5 µg/ml) ou son excipient (DMSO) pour disposer d'un contrôle positif de cytotoxicité.

b) Test MTS

Ce test colorimétrique mesure la capacité des mitochondries des cellules vivantes à oxyder le sel de tétrazolium MTS en formazan. Le produit coloré formé est proportionnel à l'activité déshydrogénase des mitochondries. La mesure de l'absorbance permet donc de quantifier la viabilité cellulaire. Après 24, 48, et 72 heures d'incubation dans les conditions décrites ci- dessus, les cellules sont lavées avec du milieu complet et incubées 1 heure à 37°C en présence de 100 µl de réactif MTS contenant 48µg/ml de PMS et 2mg/ml de MTS. La mesure d'absorbance à 490 nm est réalisée par un lecteur de microplaque (MRX, Dynatech Laboratories, VWR international). Les résultats sont exprimés en pourcentage d'activité MTS par rapport aux conditions contrôles (cellules cultivées en absence d'hydrogel).

c) Dénombrement cellulaire

Après 24, 48 et 72 heures d'incubation dans les conditions précisées ci-dessus, le milieu de culture avec ou sans hydrogel est aspiré et remplacé par 200 µl de trypsine/EDTA. Les cellules sont incubées 2 minutes en atmosphère humide à 37°C et 5 % $CO_2$. Les cellules sont ensuite collectées et centrifugées (8 min, 1200 rpm) en présence de 2 ml de milieu de culture complet. Le culot est resuspendu dans 2 ml de milieu de culture complet et les cellules sont dénombrées après coloration vitale par une solution de Bleu Trypan (0.04% dans PBS) sur cellule de Malassez. Les résultats sont exprimés en nombre total de cellules par puits.

<u>**RESULTATS**</u>

**2.3 Caractérisation des Chondrocytes nasaux humains**

Afin de caractériser le phénotype des cellules isolées de cartilage nasal humain, les inventeurs ont étudié par RT-PCR l'expression de certains marqueurs du phénotype chondrocytaire après isolation et au cours d'une culture en monocouche. Cette étude montre que les chondrocytes nasaux fraîchement isolés (P0) exprimaient le collagène II, l'Aggrécane, et le collagène X mais également le collagène I. Lors des différents passages, les inventeurs ont observé une modification de l'expression de ces différents gènes. L'expression du collagène de type II a diminué entre P0 et P3. L'aggrécane a présenté une baisse de son expression entre P0 et P1. Le collagène X était faiblement exprimé par les chondrocytes fraîchement isolés, son expression n'était plus détectable à P3. En revanche, l'expression du collagène de type I a augmenté fortement entre P2 et P3.

Ces résultats montrent que les chondrocytes nasaux fraîchement isolés exprimaient le collagène II, l'aggrécane et le collagène X. L'expression de ces marqueurs chondrocytaires a diminué en culture monocouche et s'est accompagnée d'une forte augmentation de l'expression du collagène de type 1.

### 2.4 Cytotoxicité de l'hydrogel

a) Mesure de l'activité MTS des chondrocytes cultivés au contact du gel :

Pour déterminer l'effet cytotoxique de l'hydrogel sur les chondrocytes, les inventeurs ont réalisé un dosage de l'activité MTS sur les lignées cellulaires SW1353 et C28/I2 et sur les chondrocytes humains nasaux après 24h, 48h et 72 heures de culture au contact de l'hydrogel Si-HPMC.

Les résultats ont montré que l'activité MTS n'était pas modifiée par la présence de l'hydrogel. En effet, il n'a pas été observé de différence significative, pour les chondrocytes SW1353, C28/I2 et les chondrocytes nasaux humains, entre les conditions de cultures contrôles et les cultures réalisées au contact de l'hydrogel. En revanche, l'actinomycine-D, inhibiteur de la transcription, utilisée ici comme contrôle positif de la cytotoxicité a entraîné une diminution significative de l'activité MTS dès 24 heures. L'activité MTS des chondrocytes SW1353, C28I2 et isolés du cartilage nasal humain après 24 heures en présence d'actinomycine D a diminué respectivement de 72%, 69% et 86% (figures 1A à 1C).

Ces résultats montrent que l'hydrogel n'a pas affecté l'activité MTS ni des chondrocytes SW1353 et C28/I2, ni des chondrocytes nasaux humains.

b) Dénombrement au bleu trypan:

Afin de déterminer l'influence de l'hydrogel sur la prolifération cellulaire, les inventeurs ont réalisé un dénombrement cellulaire à l'aide d'une coloration au bleu trypan. Cette coloration permet de distinguer les cellules vivantes (blanches) des cellules mortes (bleues). Ce dénombrement est effectué après 24h, 48h et 72 heures de culture au contact de l'hydrogel pour les lignées cellulaires SW1353 et C28/I2 et pour les chondrocytes nasaux humains (figures 2a à 2C).

Il n'a pas été observé de différence significative entre le nombre de chondrocytes SW1353, C28/I2 et chondrocytes nasaux humains cultivés dans les conditions contrôles et lors d'une culture réalisée au contact de l'hydrogel de Si-HPMC. En revanche, l'actinomycine-D a entraîné une diminution significative du nombre de cellules dès 24 heures. Le nombre de chondrocytes SW1353 a diminué de 88%, le nombre de chondrocytes C28/I2 a diminué de 81% après 24 heures d'incubation avec l'actinomycine D. Les chondrocytes nasaux humains ont enregistré une diminution de 43% après 24h de traitement par l'actinomycine D.

Ces résultats montrent que l'hydrogel n'a pas entraîné de modification de la prolifération que ce soit pour les chondrocytes SW1353, les chondrocytes C28/12 ou les chondrocytes nasaux humains.

## Exemple 3 : Culture des chondrocytes dans l'hydrogel

### 3.1 Observation des chondrocytes en culture tridimensionnelle

**MATERIELS ET METHODES**

Conditions expérimentales

Matériels:

- Cell tracker green (CTG) (Molecular probes, Leiden, The Netherlands).
- Ethidium Homodimer-1 (EthD-1) ( Molecular probes) .

Les chondrocytes articulaires de lapins, les cellules SW1353 et les C28/I2, sont mis en suspension dans l'hydrogel à raison de 1 million de cellules/ml d'hydrogel. 500 µl de ce mélange sont déposés dans des puits de plaques de culture 24 puits. Les cellules sont ensuite cultivées en présence de milieu complet pendant 48h, 96 h, 1 semaine et deux semaines. Parallèlement, un contrôle positif de mort cellulaire est réalisé par addition d'une solution d'actinomycine D à 5µg/ml. Les cellules cultivées au sein de l'hydrogel sont observées grâce au cell tracker green (CTG) et ethidium homodimer1 (EthD-1). Le CTG est un produit incolore qui diffuse librement au travers des membranes cellulaires. Il est ensuite transformé dans le cytoplasme par la glutathione S-transferase en un produit fluorescent incapable de ressortir de la cellule. Le CTG produit une fluorescence verte dans les cellules vivantes. L'EthD-1 est un agent intercalant de l'ADN dont la fluorescence augmente de 40 fois après fixation sur les acides nucléiques. Il ne pénètre que dans les cellules présentant des dommages membranaires. L'emploi conjoint de ces deux colorants permet une colocalisation des cellules vivantes (coloration verte) et des cellules mortes (coloration rouge) (Magne et al., 2003). Le milieu de culture est aspiré et remplacé par 400 µl d'une solution de CTG à 5 µM dans du milieu de culture complet. Les cellules sont ensuite incubées 1h à 37°C. La solution de CTG est ensuite remplacée par du milieu complet et les cellules sont à nouveau incubées 30 minutes à 37°C. Après un rinçage au PBS, les cellules sont traitées 1 h à température ambiante et à l'abri de la lumière par 400 µl d'une solution d'EthD-1 à 1µM dans du milieu complet sans SVF. Le milieu est finalement enlevé, l'hydrogel est aspiré et monté entre lame et lamelle. Les images sont obtenues par microscopie épifluorescente (Axioplan, Zeiss, lena, Germany) et sont enregistrées à l'aide d'une caméra numérique DC30 (Kappa opto-electronics Gmbh, Gleichen, Germany).

Les cellules dans le gel ont également été visualisées par microscopie confocale inversée à balayage laser (IFR 26, Caroline Vignes-Colombeix) après 24, 48 et 72 h de culture comme détaillé ci-dessus. Le microscope confocale crée une image nette en éliminant les signaux n'appartenant pas au plan focal. Ce microscope possède une platine porte-objet pouvant se déplacer dans l'axe Z, ce qui permet de faire varier le plan focal et ainsi de visualiser différents plans de l'échantillon. La fluorescence du CTG a été repérée en utilisant le détecteur de la fluorescéine isothiocyanate (FITC: λex = 488 nm; λem collectée = 490-560 nm). La fluorescence de EthD-1 est visualisée par le détecteur de la rhodamine (TRITC: λex = 568 nm; λem collectée = 570-700 nm).

**<u>RESULTATS</u>**

Dans le but d'observer le développement des chondrocytes lors d'une culture tridimensionnelle au sein de l'hydrogel, les inventeurs ont effectué des colorations (CTG et EthD-1) sur les chondrocytes SW1353, C28/12 et sur des chondrocytes articulaires de lapin. Ils ont ensuite observé les chondrocytes en microscopie à fluorescence. Au sein de l'hydrogel, les chondrocytes SW1353, les C28/I2 ou les chondrocytes articulaires de lapin ont présenté une forme arrondie et étaient fortement colorés en vert par le cell tracker green. De rares cellules apparaissaient colorées en rouge. Les chondrocytes SW1353, C28I2 et articulaires de lapins se sont développés en trois dimensions dans l'hydrogel en formant des nodules dont le nombre et la taille augmentaient en fonction du temps de culture.

Afin de proposer une observation tridimensionnelle des cellules cultivées dans l'hydrogel, ils ont complété leurs observations par de la microscopie confocale. Seules les observations des cellules C28I2 sont reportées dans ce travail. La microscopie confocale a montré les mêmes résultats que ceux obtenus précédemment en microscopie fluorescente classique. Les cellules se développent sous forme de nodules. Les cellules traitées à l'actinomycine D pendant 48h ont présenté une forte coloration rouge. De rares cellules conservaient une coloration verte. Ces résultats indiquent que dans nos conditions de culture, la double coloration cell tracker green/ethydium homodimer-1 a permis de différencier les cellules vivantes des cellules mortes. Les observations des chondrocytes SW1353, des chondrocytes nasaux humains et des chondrocytes articulaires de lapin sont en cours d'analyse.

L'ensemble de ces résultats montrent que les chondrocytes SW1353, C28/12 et les chondrocytes articulaires de lapin ont conservé leur viabilité en culture tridimensionnelle au sein de l'hydrogel.

**3.2 Analyse du phénotype chondrocytaire**

Afin d'analyser le phénotype chondrocytaire, l'expression des messagers codant pour certains marqueurs des chondrocytes a été recherchée par RT-PCR.

a) <u>Conditions expérimentales</u>

Matériels :

- Trizol® reagent (Invitrogen Corporation).

Afin de caractériser le phénotype des cellules isolées du cartilage nasal humain, une fraction des cellules fraîchement isolées est congelée en présence de Trizol® (passage 0; P0). L'autre fraction cellulaire est répartie à raison de 10 000 cellules/$cm^2$ dans des flacons de 25 $cm^2$. Les cellules sont repiquées toutes les semaines dans les conditions décrites précédemment (chapitre a)). Après 1, 2 et 3 passages, les cellules sont congelées à -80°C en présence de Trizol® (Passage 1 (P1), 2 (P2), et 3 (P3)).

Les chondrocytes fraîchement isolés de cartilage articulaire de lapins sont répartis à raison de 1 million de cellules/ml d'hydrogel et 2ml de ce mélange sont déposés dans des puits de plaque de culture 6 puits. Les cellules sont ensuite cultivées pendant 3 semaines avant congélation en présence de Trizol®.

b) <u>Extractions des ARN totaux</u>

Matériels :

- Chloroforme (VWR).
- Isopropanol (Sigma Aldrich).
- Ethanol (VWR).
- Agarose (Promega Corporation)
- bromure d'ethidium (BET) (Promega corporation)
- Tris Borate EDTA (TBE) (Invitrogen corporation)

Les cellules sont décongelées sur de la glace, grattées puis la solution de Trizol® est collectée et centrifugée pendant 10 minutes à 12 000 rpm et à 4°C. La phase supérieure est prélevée et additionnée de 200 µl de chloroforme puis vortexée 15 secondes. Après 10 minutes d'incubation à température ambiante, les échantillons sont centrifugés 15 minutes à 12 000 rpm et à 4°C. La phase aqueuse est prélevée et les ARNs totaux sont précipités par centrifugation (12000 rpm, 15 minutes, 4°C) en présence de 500 µl d'isopropanol. Le surnageant est prélevé et le culot est lavé avec de l'éthanol 75 % puis séché. Les ARNs totaux sont repris dans 20 µl d'eau. La quantité d'ARNs isolée est ensuite évaluée par mesure de l'absorbance à 260 nm. Les concentrations d'ARNs sont finalement ajustées à 1 µg/µl. Afin de vérifier l'intégrité des ARNs extraits, 500 ng d'ARNs totaux sont séparés par électrophorèse sur gel d'Agarose à 1% dans du tampon TBE contenant du BET. La migration s'effectue à 100V pendant 30 minutes. Les ARNs totaux sont visualisés à l'aide d'un transilluminateur UV.

c) <u>Analyse des transcripts par RT-PCR</u>

- Traitement à la DNAase

Matériels :

- Désoxyribonucléase I (DNAse I) 5U/µl (Invitrogen Corporation)
- Tampon de DNAse I 10X (Invitrogen Corporation)
- Acide Ethylène diamine tétra acétique (EDTA) 25 mM (Invitrogen Corporation).

Deux µg d'ARN sont prélevés et additionnés d'1 µl de tampon de DNAse I 10X, 1 µl de DNAse I (5U/µl) et le volume est ajusté à 10µl avec de l'eau stérile. Les échantillons sont ensuite incubés 15 minutes à température ambiante. La réaction est arrêtée par l'addition de 1µl d'EDTA 25 mM et le produit de la réaction est placé 10 minutes à 65°C.

- Transcription inverse (RT)

Matériels:

- Désoxynucleotide triphosphate (dNTP) 10 mM (Invitrogen Corporation).
- Amorces hexanucléotidiques à 0.5µg/µl (Random Hexamers, Promega Corporation hexaprimer C1181).
- Avian Myeloblastosis virus reverse transcriptase (AMV-RT) 10U/µl (Promega Corporation).
- Tampon AMV-RT 5X (Promega corporation)
- RNAse inhibitor ( RNAsine) 400U/µl (Promega Corporation).

A chaque échantillon préalablement traité à la DNAase, sont ajoutés 3µl de dNTP à 10 mM, 3µl d'amorces hexanucléotidiques, 6µl de tampon AMV-RT , 1µl d'AMV-RT, 1µl de RNAsine et le volume est ajusté à 30 µl avec de l'eau stérile. La transcription inverse est réalisée à l'aide d'un thermocycleur (Eppendorf Mastercycler,VWR) dans les conditions suivantes : 10 minutes à 25°C, 50 minutes à 42°C et 10 minutes à 95°C.La solution d'ADN complémentaire

simple brin (ADNc) ainsi obtenue est ensuite stockée à -20°C jusqu'à son utilisation pour la réaction de PCR.

- Réaction de polymérase en chaîne (PCR)

Matériels :

- Amorces oligomériques spécifiques (MWG Biotech, Courtaboeuf, France).
- Tampon Taq polymérase 10X (Invitrogen Corporation).
- Taq polymérase 5U/μl (Invitrogen Corporation).
- MgCl2 50 mM (Invitrogen Corporation).

La PCR est réalisée à partir de 2 μl de solution de cDNA auxquels sont ajoutés 0,5 μl d'amorce sens et 0,5 μl d'amorces antisens à 100ng/μl, 5 μl de tampon de taq polymérase, 1.5 μl de MgCl2 , 0,5 μl de taq polymerase et 40 μl d'eau. Les réactions de PCR sont réalisées à l'aide d'un thermocycleur (eppendorf Mastercycler) dans les conditions suivantes : 3 minutes de dénaturation à 94°C suivi de 30 cycles de 20 secondes à 94°C (dénaturation), 20 secondes à 60°C (hybridation) et 20 secondes d'élongation à 72°C. La réaction de PCR est achevée par 10 minutes d'élongation à 72°C. Les résultats de cette étude présentent des produits de PCR obtenus dans la phase exponentielle d'amplification. La séquence des amorces, les températures d'hybridation et la taille des amplicons sont précisées dans le tableau 2 ci dessous.

Tableau 2 : Séquence des amorces, température d'hybridation et taille des amplicons pour les gènes référencés.

| Gènes | Séquences des amorces Sens (S) et anti-sens (AS) | Tm | Taille amplicons |
|---|---|---|---|
| actine humaine | S 5'-TCTCCATGTCGTCCCAGTTG-3'<br>AS 5'-AGTCTTCCCCTCCATCGTTG-3' | 60°C | 164 pb |
| Collagène II (alpha 1) humain et lapin | S 5'-GGCAATAGCAGGTTCACGTACA-3'<br>AS 5'-GAGGCGTGAGGTCTTCTGTGA-3' | 60°C | 108 pb |
| Aggrécane humain et lapin | S 5'-CCCTGGCAATGATGGCACTGTTC-3'<br>AS 5'-TGGCAATGATGGCACTGTTC-3' | 60°C | 117 pb |
| Collagène I (alpha 2) humain | S 5'-CATGGAAACCGTGGTCAAACT-3'<br>AS 5'-ACCAGCGATACCAGGCAGAC-3' | 60°C | 186 pb |
| Collagène X humain et lapin | S 5'-CAAGGCACCATCTCCAGGAA-3'<br>AS 5'-GCATTTGGTATCGTTCAGCGT-3' | 60°C | 131 pb |
| Collagène I (alpha 1) lapin | S 5'-GATGCGTTCCAGTTCGAGTA-3'<br>AS 5'- GGTCTTCCGGTGGTCTTGTA-3' | 55°C | 312 pb |

d) <u>Séparation sur gel d'agarose</u>

La séparation électrophorétique des produits d'amplification PCR a été réalisée sur gel d'agarose à 2% dans du tampon Tris Borate EDTA (TEB) 1X. Les bandes sont révélées par du bromure d'éthidium à l'aide d'un trans-illuminateur UV. La taille des amplicons est référencée dans le tableau 2.

d) Analyse densitométrique semi-quantitative des gels d'agarose.

L'intensité des bandes obtenues sur les gels d'agarose à 2% (gènes d'intérêts et gène de référence) a été estimée à l'aide du logiciel Leica Q500 (Leica Imagine Systems, Cambridge, UK) permettant l'analyse semi-quantitative par densitométrie des transcrits amplifiés aux différentes conditions expérimentales. Les résultats sont exprimés comme le ratio gène d'intérêt/gène de référence en unité arbitraire.

## RESULTATS

Afin de vérifier le maintien du phénotype des chondrocytes articulaires de lapin en culture tridimensionnelle au sein de l'hydrogel, ils ont évalué par RT-PCR l'expression des transcrits codant pour le collagène de type II, l'aggrécane et le collagène X. Parallèlement, l'expression du collagène de type I a également été recherchée.

Lors d'une culture en monocouche, les inventeurs ont observé une diminution de l'expression du collagène de type II, de l'aggrécane et du collagène de type X. A près trois semaines de culture le collagène de type II demeurait faiblement exprimé, alors que le collagène de type X et l'aggrécane n'étaient plus détectables. Parallèlement, nos résultats montrent que l'expression du collagène de type I est augmentée après trois semaines de culture en monocouche.

A l'inverse, lorsque les cellules sont cultivées en trois dimensions dans l'hydrogel pendant trois semaines, l'expression du collagène de type II, de l'agrécane et du collagène de type X sont maintenues. L'expression du collagène de type I n'est pas apparue stimulée.

Ces résultats indiquent que la culture tridimensionnelle des chondrocytes articulaires de lapin permet le maintien de l'expression des marqueurs chondrocytaires (collagène II, aggrécane et collagène X).

## Exemple 4 : Etude de la redifférenciation des chondrocytes en culture tridimensionnelle

Dans le but d'étudier la redifférenciation des chondrocytes en culture tridimensionnelle dans l'hydrogel préparé à l'exemple 1, des chondrocytes nasaux humains et articulaire de lapin fraîchement isolés ont été cultivés en monocouche pendant 4 semaines, puis en trois dimensions dans l'hydrogel et parallèlement en monocouche pendant 4 semaines.

Les ARN totaux ont été extrait à chaque passage lors des cultures en monocouche et à 2, 3 et 4 semaines pour les cultures tridimensionnelles. L'expression du collagène de type II et l'agrécanne marqueurs spécifiques du phénotype chondrocytaire, mais aussi le collagène de type I marqueurs de la dédifférenciation des chondrocytes ont été recherchés par RT-PCR.

Les résultats montrent que les chondrocytes fraîchement isolés expriment le collagène de type II, le collagène de type I et l'agrécanne. L'expression de ces marqueurs est modifiée lors de la culture en monocouche, l'expression du collagène II et de l'agrécanne diminue lors des différents passages jusqu'à disparaître au bout de trois passages, alors que l'expression du collagène de type I augmente fortement des le premier passage et reste fortement exprimé lors des différents passages. Les chondrocytes semblent donc s'être dédifférenciés au bout de trois passages en culture monocouche. Ensuite, lors de la culture tridimensionnelle de ces chondrocytes dans l'hydrogel, les résultats ont montré une augmentation de l'expression du collagène de type II ainsi que de l'agrécanne mais également une diminution de l'expression du collagène de type I. Ces résultats suggèrent que les cellules ont retrouvé un phénotype chondrocytaire dans l'hydrogel.

Ces résultats indiquent que l'hydrogel utilisé permet la redifférenciation de chondrocytes préalablement dédifférenciés.

## Tests statistiques

Toutes les expériences ont été réalisées en triple. Chaque expérience a été réalisée deux fois. Les résultats sont exprimés comme la moyenne +/- l'écart type. Les études comparatives de moyennes ont été effectuées en utilisant le test Anova. Les résultats sont considérés comme significativement différents pour $p<0,05$.

## BIBLIOGRAPHIE

- Bourges et al., 2002a, General properties of silated hydroxyethylcellulose for potential biomedical applications, Biopolymers **63**(4) :232-8
- Bourges X, Weiss P, Daculsi G, Legeay G, 2002b Synthesis and general properties of silated-hydroxypropyl methylcellulose in prospect of biomedical use. Adv Colloid Interface Sci **99**(3):215-28

- Brittberg M, Lindahl A, Nilsson A, Ohlsson C, Isaksson O, Peterson L 1994 Treatment of deep cartilage defects in the knee with autologous chondrocyte transplantation. N Engl J Med **331**(14):889-95
- Cancedda R, Dozin B, Giannoni P, Quarto R 2003 Tissue engineering and cell therapy of cartilage and bone. Matrix Biol **22**(1):81-91
- Ghayor C, Herrouin JF, Chadjichristos C, Ala-Kokko L, Takigawa M, Pujol JP, Galera P 2000 Regulation of human COL2A1 gene expression in chondrocytes. Identification of C-Krox-responsive elements and modulation by phenotype alteration. J Biol Chem **275**(35):27421-38
- Goldring MB, Birkhead JR, Suen LF, Yamin R, Mizuno S, Glowacki J, Arbiser JL, Apperley JF 1994 Interleukin-1 beta-modulated gene expression in immortalized human chondrocytes. J Clin Invest **94**(6):2307-16
- Hunziker EB 2002 Articular cartilage repair: basic science and clinical progress. A review of the current status and prospects. Osteoarthritis Cartilage **10**(6):432-63
- Magne D, Bluteau G, Faucheux C, Palmer G, Vignes-Colombeix C, Pilet P, Rouillon T, Caverzasio J, Weiss P, Daculsi G, Guicheux J 2003 Phosphate is a specific signal for ATDC5 chondrocyte maturation and apoptosis-associated mineralization : possible implication of apoptosis in the regulation of endochondral ossification. J Bone Miner Res **18**(8):1430-42
- Mengshol JA, Vincenti MP, Coon CI, Barchowsky A, Brinckerhoff CE 2000 Interleukin-1 induction of collagenase 3 (matrix metalloproteinase 13) gene expression in chondrocytes requires p38, c-Jun N-terminal kinase, and nuclear factor kappaB: differential regulation of collagenase 1 and collagenase 3. Arthritis Rheum **43**(4):801-11
- Shapiro F, Koide S, Glimcher MJ 1993 Cell origin and differentiation in the repair of full-thickness defects of articular cartilage. J Bone Joint Surg Am **75**(4):532-53

## Revendications

**1.** Utilisation d'un hydrogel d'hydroxyéthylcellulose (HEC) silanisée ou d'hydroxypropylméthylcellulose (HPMC) silanisée, auto-réticulant en fonction du pH, pour la culture tridimensionnelle *ex vivo* de chondrocytes.

**2.** Utilisation selon la revendication 1, dans laquelle l'hydrogel est susceptible d'être obtenu par réaction d'HEC ou d'HPMC avec un composé de formule (1) :

$$X\ Si(OZ)_3 \qquad (1)$$

où X représente un atome d'halogène ou un groupe hydrocarboné à fonction époxy, notamment en $C_{2-20}$, et Z est choisi parmi un atome d'hydrogène, un métal alcalin et un groupe alkyle, notamment en $C_{1-5}$.

**3.** Utilisation selon l'une des revendications 1 ou 2, dans laquelle l'HEC ou l'HPMC porte des groupes latéraux silanolate ou précurseurs de silanolate de métal alcalin ou d'ammonium représentant de 0,5 à 5% du poids sec total de l'HEC ou l'HPMC.

**4.** Utilisation selon l'une des revendications 1 à 3, l'hydrogel étant constitué d'un polymère de formule simplifiée :

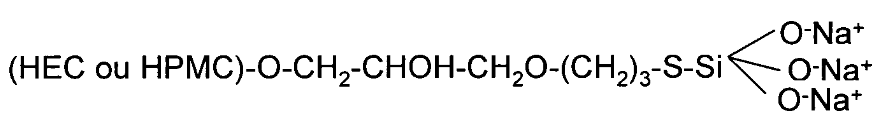

**5.** Procédé *ex vivo* de préparation d'un complexe de cellules intégrées dans un hydrogel, complexe destiné à être injecté dans un site cartilagineux, ledit procédé comprenant le mélange *ex vivo* de chondrocytes avec un hydrogel d'hydroxyéthylcellulose (HEC) silanisée ou d'hydroxypropylméthylcellulose (HPMC) silanisée, autoréticulant en fonction du pH, dans un tampon biologique au pH approprié pour la réticulation de l'hydrogel, dans des conditions et une durée appropriées pour l'intégration et la culture tridimensionnelle des chondrocytes dans l'hydrogel.

**6.** Procédé selon la revendication 5, comprenant les étapes ex vivo suivantes :

- l'amplification de chondrocytes en monocouche, sur un support solide ;
- la récolte des chondrocytes amplifiés, dédifférenciés de par leur amplification en monocouche ;
- le mélange des chondrocytes amplifiés dédifférenciés avec l'hydrogel dans un tampon biologique au pH approprié pour la réticulation de l'hydrogel, ce qui conduit à l'intégration des chondrocytes au sein de l'hydrogel et à leur redifférenciation.

**7.** Procédé selon la revendication 5 ou 6, dans lequel l'hydrogel est susceptible d'être obtenu par réaction d'HEC ou d'HPMC avec un composé de formule (1) :

$$X\ Si(OZ)_3 \qquad (1)$$

où X représente un atome d'halogène ou un groupe hydrocarboné à fonction époxy, notamment en $C_{2-20}$, et Z est choisi parmi un atome d'hydrogène, un métal alcalin et un groupe alkyle, notamment en $C_{1-5}$.

**8.** Procédé selon l'une des revendications 5 à 7, dans lequel l'HEC ou l'HPMC porte des groupes latéraux silanolate ou précurseurs de silanolate de métal alcalin ou d'ammonium représentant de 0,5 à 5% du poids sec total de l'HEC ou l'HPMC.

**9.** Procédé selon l'une des revendications 5 à 8, l'hydrogel étant constitué d'un polymère de formule simplifiée :

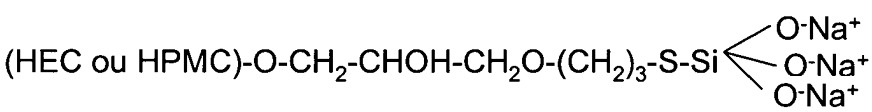

FIG.1

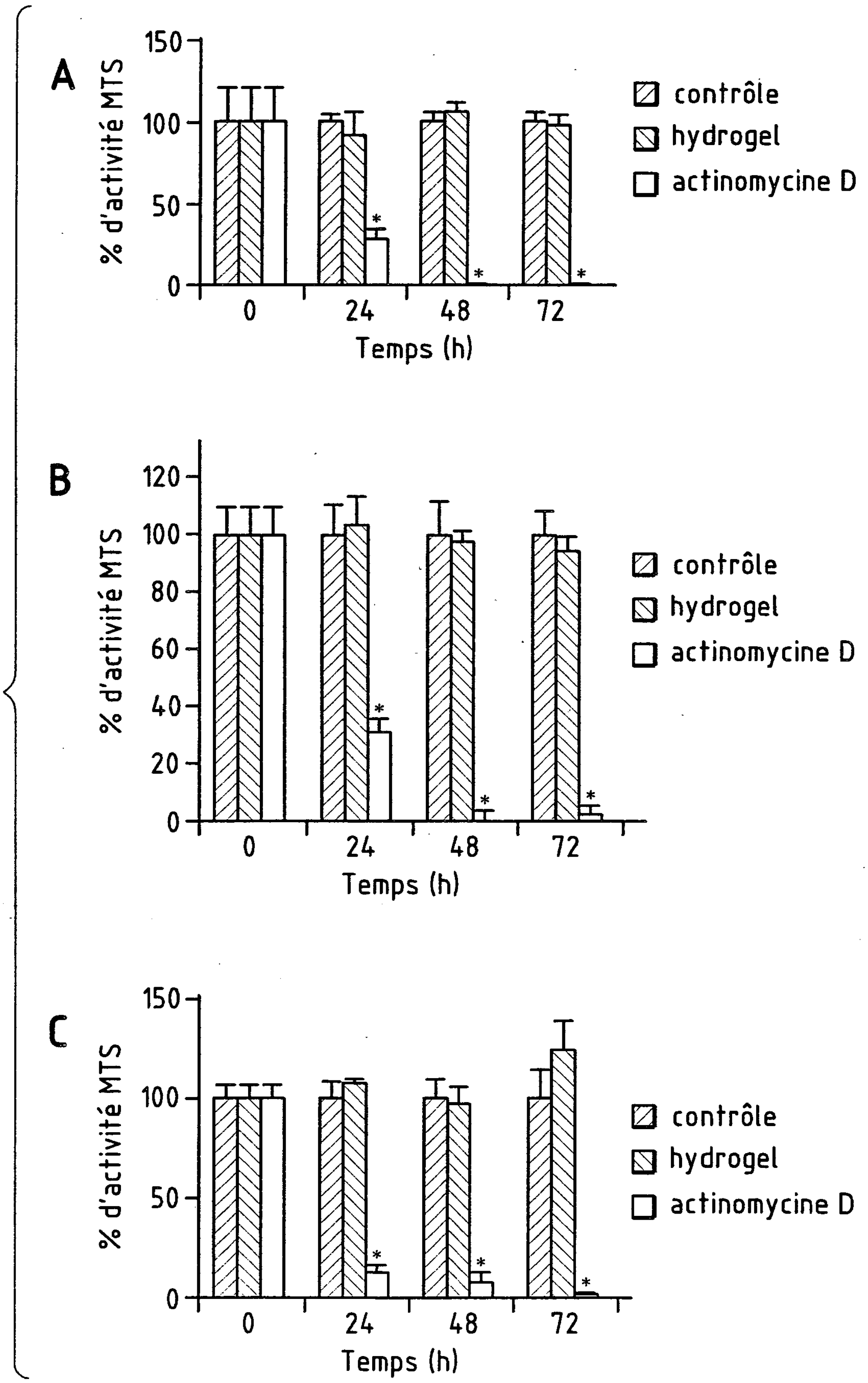
A
150
100
50
0
% d'activité MTS
0
24
48
72
Temps (h)
contrôle
hydrogel
actinomycine D
B
120
100
80
60
40
20
0
% d'activité MTS
0
24
48
72
Temps (h)
contrôle
hydrogel
actinomycine D
C
150
100
50
0
% d'activité MTS
0
24
48
72
Temps (h)
contrôle
hydrogel
actinomycine D

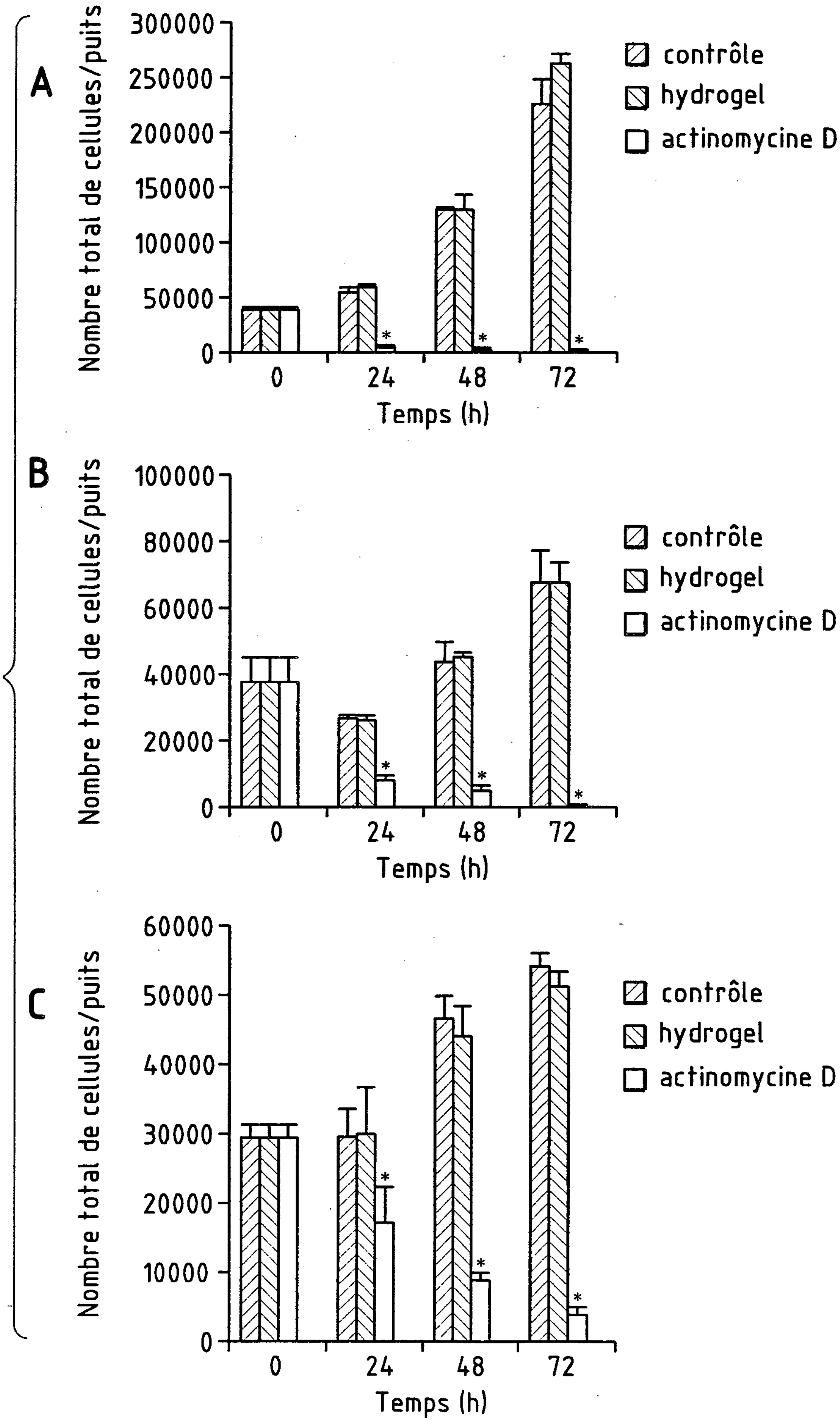
A
Nombre total de cellules/puits
300000
250000
200000
150000
100000
50000
0
0
24
48
72
Temps (h)
contrôle
hydrogel
actinomycine D
*
B
Nombre total de cellules/puits
100000
80000
60000
40000
20000
0
0
24
48
72
Temps (h)
contrôle
hydrogel
actinomycine D
C
Nombre total de cellules/puits
60000
50000
40000
30000
20000
10000
0
0
24
48
72
Temps (h)
contrôle
hydrogel
actinomycine D

FIG.2

Office européen des brevets

**RAPPORT DE RECHERCHE EUROPEENNE**

Numéro de la demande
EP 03 29 2759

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A,D | WO 97 05911 A (DACULSI GUY ;DUPRAZ ANNE (FR); WEISS PIERRE (FR); CENTRE NAT RECH) 20 février 1997 (1997-02-20)<br>* le document en entier *<br>--- | 1-9 | A61L27/46<br>A61L27/50 |
| A,D | US 6 129 761 A (HUBBELL JEFFREY A) 10 octobre 2000 (2000-10-10)<br>* le document en entier *<br>----- | 1-9 | |
| | | | DOMAINES TECHNIQUES RECHERCHES (Int.Cl.7) |
| | | | A61L |

Le présent rapport a été établi pour toutes les revendications

1

| Lieu de la recherche | Date d'achèvement de la recherche | Examinateur |
|---|---|---|
| MUNICH | 1 avril 2004 | Trifilieff-Riolo, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 03 29 2759

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-04-2004

| Document brevet cité au rapport de recherche | | Date de publication | Membre(s) de la famille de brevet(s) | | | Date de publication |
|---|---|---|---|---|---|---|
| WO 9705911 | A | 20-02-1997 | FR | 2737663 | A1 | 14-02-1997 |
| | | | AT | 214292 | T | 15-03-2002 |
| | | | AU | 707039 | B2 | 01-07-1999 |
| | | | AU | 6704296 | A | 05-03-1997 |
| | | | CA | 2226304 | A1 | 20-02-1997 |
| | | | DE | 69619830 | D1 | 18-04-2002 |
| | | | DE | 69619830 | T2 | 12-09-2002 |
| | | | DK | 843562 | T3 | 21-05-2002 |
| | | | EP | 0843562 | A1 | 27-05-1998 |
| | | | ES | 2173306 | T3 | 16-10-2002 |
| | | | WO | 9705911 | A1 | 20-02-1997 |
| | | | JP | 11510076 | T | 07-09-1999 |
| | | | NO | 980504 | A | 06-04-1998 |
| | | | PT | 843562 | T | 30-08-2002 |
| | | | US | 6001394 | A | 14-12-1999 |
| | | | ZA | 9606635 | A | 05-02-1998 |
| US 6129761 | A | 10-10-2000 | AU | 720569 | B2 | 08-06-2000 |
| | | | AU | 6048596 | A | 30-12-1996 |
| | | | CA | 2223932 | A1 | 19-12-1996 |
| | | | EP | 0835143 | A1 | 15-04-1998 |
| | | | JP | 11505734 | T | 25-05-1999 |
| | | | WO | 9640304 | A1 | 19-12-1996 |

EPO FORM P0460

Pour tout renseignement concernant cette annexe : voir Journal Officiel de l'Office européen des brevets, No.12/82